**Europäisches Patentamt**

(19) **European Patent Office**

**Office européen des brevets**

(11) Veröffentlichungsnummer : **0 234 403 B1**

(12) # EUROPÄISCHE PATENTSCHRIFT

(45) Veröffentlichungstag der Patentschrift :
18.09.91 Patentblatt 91/38

(51) Int. Cl.⁵ : **A61F 13/04**

(21) Anmeldenummer : **87101829.7**

(22) Anmeldetag : **10.02.87**

(54) **Laminatverband.**

(30) Priorität : **19.02.86 DE 3605198**

(43) Veröffentlichungstag der Anmeldung :
**02.09.87 Patentblatt 87/36**

(45) Bekanntmachung des Hinweises auf die
Patenterteilung :
**18.09.91 Patentblatt 91/38**

(84) Benannte Vertragsstaaten :
**AT BE CH DE ES FR GB GR IT LI LU NL SE**

(56) Entgegenhaltungen :
EP-A- 0 128 399
EP-A- 0 136 021
GB-A- 807 406
US-A- 4 570 622

(73) Patentinhaber : **BAYER AG**
**W-5090 Leverkusen 1 Bayerwerk (DE)**

(72) Erfinder : **Richter, Roland, Dr.**
**Roggendorfstrasse 53**
**W-5000 Köln 80 (DE)**
Erfinder : **Mayer, Wolfram, Dr.**
**Auf dem Heidchen 17**
**W-5068 Odenthal (DE)**
Erfinder : **Müller, Hanns Peter, Dr.**
**Im Kerberich 6**
**W-5068 Odenthal (DE)**
Erfinder : **Schäpel, Dietmar, Dr.**
**Johanniterstrasse 15**
**W-5000 Köln 80 (DE)**
Erfinder : **Strache, Dietmar, Dr.**
**Am Walde 11**
**W-3220 Alfeld (DE)**

## Beschreibung

Die Erfindung betrifft Laminatverbände für den medizinischen oder tiermedizinischen Einsatz.

Die Verwendung von mit Gips imprägnierten Binden als versteifendes Verbandsmaterial ist bekannt. Derartige Gipsverbände sind unerwünscht schwer, wenig luftdurchlässig, verlieren in feuchtem Zustand, beispielsweise durch Einwirkung von Wasser auf den ausgehärteten Verband, rasch an Festigkeit, sie behindern wegen ihrer Röntgenabsorption und -streuung die diagnostische Auswertung von Röntgenaufnahmen und sind wegen ihrer mangelhaften Wasserfestigkeit oft Anlaß zu Hautirritationen, hervorgerufen durch Bakterien- oder Pilzbewuchs im Verband.

Ebenfalls bekannt sind Stützverbände aus Kunststoffmaterial, bei denen ein textiles oder auf Glasfasern basierendes Trägermaterial mit einem Polymer, beispielsweise einem in Gegenwart von Wasser aushärtenden Polyurethan, beschichtet ist (Chemical Orthopaedices and Related Research 103, 109-117 (1974) und DE-OS 2 357 931 und US Patente 4 376 438, 4 411 262, 4 502 479 und 4 570 622). Die Stützverbände auf Basis von Kunststoffmaterialien weisen nur eine geringe Modellierbarkeit auf.

Es wurden Laminatverbände gefunden, die in getrennten Lagen Gips und ein durch Wasser ausgehärtetes Kunststoffharz, jeweils auf einem Trägermaterial, enthalten, wobei zumindest die Gipslagen mit einer Kunststoffdispersion ausgerüstet sind.

Die erfindungsgemäßen Laminatverbände weisen neben einer hohen Bruchstabilität eine hohe Modellierbarkeit auf.

Gips für die erfindungsgemäßen Laminatverbände kann dehydratisiertes (z.B. Anhydrid) oder ganz- oder teilweise hydratisiertes Kalziumsulfat sein, das für die Verarbeitung auf dem Trägermaterial zu Gipsbänden noch Zuschläge enthalten kann.

Die Zuschläge sollen beispielsweise die Haftung auf dem textilen Träger verbessern und bei dem ausgehärteten Gips die Wasserfestigkeit erhöhen. Im allgemeinen werden die Zuschläge zusammen mit dem Kalziumsulfat bei der Herstellung der Gipsbinden eingearbeitet.

Zuschläge können beispielsweise anorganische Salze wie Kieselgur, Kreide, Alumosilikate und Kaoline oder organische Kunststoffe wie Alkylarylsulfonate, ligninsulfonsaure Salze, Melaminharze oder Cellulose- und Stärkeether (Ullmann 1976, Bd. 12, Seite 306) sein.

Die genannten Gipsbinden sind an sich bekannt.

Kunststoffharze, die in Wasser aushärten, sind bevorzugt Harze auf Polyurethan- und Polyvinyl-Basis.

Als wasserhärtende Polyurethane kommen erfindungsgemäß alle an sich bekannten organischen Polyisocyanate in Frage, d.h. beliebige Verbindungen bzw. Gemische von Verbindungen, die pro Molekül mindestens zwei organisch gebundene Isocyanatgruppen aufweisen. Hierzu gehören sowohl niedermolekulare Polyisocyanate mit einem unter 400 liegendem Molekulargewicht als auch Modifizierungsprodukte derartiger niedermolekularer Polyisocyanate mit einem aus der Funktionalität und dem Gehalt an funktionellen Gruppen berechenbaren, z.B. 400 bis 10.000, vorzugsweise 600 bis 8.000, und insbesondere 800 bis 5.000, betragenden Molekulargewicht. Geeignete niedermolekulare Polyisocyanate sind beispielsweise solche der Formel

$$Q\,(NCO)_n,$$

in der

n = 2 bis 4, vorzugsweise 2 bis 3,

und

Q einen aliphatischen Kohlenwasserstoffrest mit 2 bis 18, vorzugsweise 6 bis 10 C-Atomen, einen cycloaliphatischen Kohlenwasserstoffrest mit 4 bis 15, vorzugsweise 5 bis 10 C-Atomen, einen aromatischen Kohlenwasserstoffrest mit 6 bis 15, vorzugsweise 6 bis 13 C-Atomen, oder einen araliphatischen Kohlenwasserstoffrest mit 8 bis 15, vorzugsweise 8 bis 13 C-Atomen, bedeuten.

Geeignete derartige niedermolekulare Polyisocyanate sind z.B. Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethylcyclohexan, 2,4- und 2,6-Hexahydrotoluylendiisocyanat sowie beliebige Gemische dieser Isomeren, Hexahydro-1,3- und/oder -1,4-phenylendiisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-4,4',4''-triisocyanat oder Polyphenyl- polymethylenpolyisocyanate, wie sie durch Anilin-Formaldehyd-Kondensation und anschließende Phosgenierung erhalten werden.

Geeignete höhermolekulare Polyisocyanate sind Modifizierungsprodukte derartiger einfacher Polyisocyanate, d.h. Polyisocyanate mit z.B. Isocyanurat-, Carbodiimid-, Allophanat-, Biuret- oder Uretdion-Struktureinheiten, wie sie nach an sich bekannten Verfahren des Standes der Technik aus den beispielhaft genannten einfachen Polyisocyanaten der oben genannten allgemeinen Formel hergestellt werden können. Unter den höhermolekularen, modifizierten Polyisocyanaten sind insbesondere die aus der Polyurethanchemie bekannten Prepolymeren mit endständigen Isocyanatgruppen des Molekulargewichtsbereichs 400 bis 10.000, vorzugsweise 600 bis 8.000 und insbesondere 800 bis 5.000, von Interesse. Diese Verbindungen werden in an sich bekannter Weise durch Umsetzung von überschüssigen Mengen an einfachen Polyisocyanaten der beispielhaft genannten Art mit organischen Verbindungen mit mindestens zwei gegenüber Isocyanatgruppen reaktionsfähigen Gruppen, insbesondere organischen Polyhydroxylverbindungen hergestellt. Geeignete derartige Polyhydroxylverbindungen sind sowohl einfache mehrwertige Alkohole wie z.B. Ethylenglykol, Trimethylolpropan, Propandiol-1,2 oder Butandiol-1,2, insbesondere jedoch höhermolekulare Polyetherpolyole und/oder Polyesterpolyole der aus der Polyurethanchemie an sich bekannten Art mit Molekulargewichten von 600 bis 8.000, vorzugsweise 800 bis 4.000, die mindestens zwei, in der Regel 2 bis 8, vorzugsweise aber 2 bis 4 primäre und/oder sekundäre Hydroxylgruppen aufweisen. Selbstverständlich können auch solche NCO-Prepolymere eingesetzt werden, die beispielsweise aus niedermolekularen Polyisocyanaten der beispielhaft genannten Art und weniger bevorzugten Verbindungen mit gegenüber Isocyanatgruppen reaktionsfähigen Gruppen wie z.B. Polythioetherpolyolen, Hydroxylgruppen aufweisenden Polyacetalen, Polyhydroxypolycarbonaten, Hydroxylgruppen aufweisenden Polyesteramiden oder Hydroxylgruppen aufweisenden Copolymerisaten olefinisch ungesättigter Verbindungen erhalten worden sind. Zur Herstellung der NCO-Prepolymeren geeignete Verbindungen mit gegenüber Isocyanatgruppen reaktionsfähigen Gruppen, insbesondere Hydroxylgruppen, sind beispielsweise die in US-PS 4 218 543, Kolonne 7, Zeile 29 bis Kolonne 9, Zeile 25 beispielhaft offenbarten Verbindungen. Bei der Herstellung der NCO-Prepolymeren werden diese Verbindungen mit gegenüber Isocyanatgruppen reaktionsfähigen Gruppen mit einfachen Polyisocyanaten der oben beispielhaft genannten Art unter Einhaltung eines NCO/OH-Äquivalentverhältnisses von >1 zur Umsetzung gebracht. Die NCO-Prepolymeren weisen im allgemeinen einen NCO-Gehalt von 2,5 bis 30, vorzugsweise 6 bis 25 Gew.-% auf. Hieraus geht bereits hervor, daß im Rahmen der vorliegenden Erfindung unter "NCO-Prepolymeren" bzw. unter "Prepolymeren mit endständigen Isocyanatgruppen" sowohl die Umsetzungsprodukte als solche als auch ihre Gemische mit überschüssigen Mengen an nicht umgesetzten Ausgangspolyisocyanaten, die oft auch als "Semiprepolymer" bezeichnet werden, zu verstehen sind.

Erfindungsgemäß besonders bevorzugte Polyisocyanatkomponenten sind die in der Polyurethanchemie üblichen technischen Polyisocyanate, d.h. Hexamethylendiisocyanat, 1-Isocyanato-3,3,5-trimethyl-5-isocyanatomethyl-cyclohexan (Isophorondiisocyanat, abgekürzt: IPDI), 4,4'-Diisocyanato-dicyclohexylmethan, 4,4'-Diisocyanatodiphenylmethan, dessen Gemische mit den entsprechenden 2,4'- und 2,2'-Isomeren, Polyisocyanatgemische der Diphenylmethanreihe wie sie durch Phosgenierung von Anilin/Formaldehyd-Kondensaten in an sich bekannter Weise gewonnen werden können, die Biuret- oder Isocyanuratgruppen aufweisenden Modifizierungsprodukte dieser technischen Polyisocyanate und insbesondere NCO-Prepolymere der genannten Art auf Basis dieser technischen Polyisocyanate einerseits und den beispielhaft genannten einfachen Polyolen und/oder Polyetherpolyolen und/oder Polyesterpolyolen andererseits, sowie beliebige Gemische derartiger Polyisocyanate. Isocyanate mit aromatisch gebundenen NCO-Gruppen sind erfindungsgemäß bevorzugt. Eine erfindungsgemäß besonders bevorzugte Polyisocyanat-Komponente stellt teilweise carbodiimidisiertes Diisocyanatodiphenylmethan dar, welches infolge Anlagerung von monomerem Diisocyanat an die Carbodiimid-Struktur auch Uretonimingruppen aufweist.

Wasserhärtende Polyvinylharze können beispielsweise Vinylverbindungen sein, die aus einem hydrophilen Prepolymer mit mehr als einer polymerisierbaren Vinylgruppe bestehen, in der ein fester, unlöslicher Vinyl-Redox Katalysator eingelagert ist, dessen einer Bestandteil von einer wasserlöslichen bzw. wasserdurchlässigen Hülle umkapselt ist. Ein solcher Redox Katalysator ist beispielsweise Natriumhydrogensulfit/Kupfer(II)sulfat, bei dem beispielsweise das Kupfersulfat mit Poly-2-hydroxyethylmethylacrylat verkapselt ist.

Kunststoff-Stützverbände auf Basis eines wasserhärtenden Polyvinylharzes werden beispielsweise in der EP 01 36 021 beschrieben.

Trägermaterialien für die Gips- und Kunststoffharzlagen für die erfindungsgemäßen Laminatverbände können die für Stützverbände üblichen Trägermaterialien sein.

Als Trägermaterialien kommen massive oder poröse Folien oder auch Schaumstoffe aus natürlichen oder synthetischen Materialien (z.B. Polyurethan) in erster Linie luftdurchlässige, flexible Flächengebilde auf textiler Basis in Betracht, vorzugsweise mit einem Flächengewicht von 20 bis 1.000 g/m², insbesondere von 30 bis 500 g/m². Als Flächengebilde seien beispielsweise genannt:

1. Textile Gewebe, Gewirke oder Gestricke eines Flächengewichts von 20 bis 200 g/m², vorzugsweise 40 bis 100 g/m², mit einer Fadenzahl von vorzugsweise 2 bis 20 Fäden je laufenden Zentimeter in der Längs- und Querrichtung. Das textile Gewebe oder Gewirke kann aus beliebigen natürlichen oder synthetischen Garnen hergestellt sein. Vorzugsweise werden jedoch Gewebe oder Gewirke eingesetzt, die aus Baum-

3

wollgarnen oder aus Mischgarnen hergestellt worden sind, die ihrerseits sowohl aus hydrophoben Fäden bzw. Fasern eines hohen E-Moduls (beispielsweise Polyester) und hydrophilen natürlichen oder synthetischen Fäden bzw. Fasern (beispielsweise Baumwolle oder Polyamid) erhalten worden sind.

2. Glasfasergewebe, -gewirke oder -gestricke eines Flächengewichts von 60 bis 500 g/m², vorzugsweise 100 bis 400 g/m², hergestellt aus Glasfasergarnen mit einem E-Modul von 7.000 bis 9.000 (daN/mm²), und einer Fadenzahl von 3 bis 10, bevorzugt 5 bis 7 in Längsrichtung und mit einer Fadenzahl von 3 bis 10, bevorzugt 4 bis 6 in Querrichtung je Zentimeter Glasfasergewebe, die durch eine spezielle Art der Hitzebehandlung eine Längenelastizität von 10 bis 30 % besitzen, sind bevorzugt. Die Gewirke können sowohl geschlichtet als auch ungeschlichtet sein.

3. Nicht gebundene oder gebundene oder genadelte Vliese auf Basis von anorganischen und vorzugsweise organischen Fasern eines Flächengewichts von 30 bis 400 g/m², vorzugsweise 50 bis 200 g/m².

Für die Herstellung erfindungsgemäßer Versteifungsverbände in Form von Schalen oder Schienen kommen auch Vliese mit Flächengewichten bis 1.000 g/m² in Betracht. Erfindungsgemäß geeignete Trägermaterialien werden beispielsweise auch in US-PS 4 134 397, US-PS 3 686 725, US-PS 3 882 857, DE-OS 3 211 634 und EP-A 61 642 beschrieben.

Kunststoffdispersionen für den erfindungsgemäßen Laminatverband sind im allgemeinen Polyurethane und/oder Polyurethanharnstoffe, die einen, die Dispergierbarkeit im Wasser gewährleistenden Gehalt an chemisch eingebauten hydrophilen Gruppen aufweisen.

Die hydrophilen Gruppen können in Form von

(a) ionischen Gruppen und/oder

(b) durch eine Neutralisationsreaktion in ionische Gruppen überführbare Gruppen und/oder

(c) Ethylenoxideinheiten ($-CH_2-CH_2-O-$) innerhalb von in das Polyurethan(harnstoff) Molekül eingebauten Polyetherketten

vorliegen.

Für die Herstellung der Kunststoffdispersionen können alle an sich bekannten, beim Auftrocknen klebfreie Filme bildenden wäßrigen Polyurethan(harnstoff)-Dispersionen verwendet werden, die - gegebenenfalls infolge eines Gehaltes an Alkoholen sowie gegebenenfalls weiteren organischen Lösungsmitteln - weitgehend unempfindlich gegenüber Koagulation durch Elektrolyten sind.

Für die Herstellung von Polyurethan(harnstoff)-Dispersionen in Wasser sind ein Reihe von Verfahren bekannt. Eine zusammenfassende Darstellung findet sich in Angw. Makr. Chem. 26, 85 bis 106 (1972), Angw. Chem. 82, 53 bis 63 (1970), J. Oil Col. Chem. Assoc. 1970, 53, 363 bis 379, Angw. Mak. Chem. 78, 133 bis 158, (1981) und in "Chemie und Technologie makromolekularer Stoffe" (29. Veröffentlichung der Fachhochschule Aachen zum 9. Colloquium am 8. Mai 1981 an der FH Aachen, Fachbereich Chemie-Ingenieurwesen).

Ein in der Praxis bevorzugtes Herstellungsverfahren für wäßrige Polyurethan(harnstoff)-Dispersionen besteht darin, daß ein in einem organischen Lösungsmittel gelöstes Isocyanatprepolymer mit einem Kettenverlängerungsmittel umgesetzt wird. Dabei enthält entweder das Prepolymer oder das Kettenverlängerungsmittel ionische oder zur Ionenbildung befähigte Gruppen. Im Laufe der Polyadditionsreaktion oder danach werden zur Ionenbildung befähigte Gruppen in ionische Gruppen überführt. Gleichzeitig oder auch anschließend erfolgt die Ausbildung der wäßrigen Dispersionen durch Zusatz von Wasser und Abdestilieren des organisches Lösungsmittels. Wie schon erwähnt, können im erfindungsgemäßen Verfahren sowohl kationische als auch anionische und nicht-ionische Polyurethandispersionen verwendet werden. Vorzugsweise werden solche wäßrige Polyurethan(harnstoff)-Dispersionen eingesetzt, welche beim Auftrocknen Polyurethanfolien mit elastischen Eigenschaften liefern. Darunter sind insbesondere gummielastische oder mindestens kerbschlagfähige Polyurethane bzw. Polyharnstoffe oder Polyhydrazodicarbonamide zu verstehen, die eine Kugeldruckhärte unter 1.400 kp/cm² (60 Sekunden nach DIN 53 456), vorzugsweise eine Shore-Härte D von weniger als 98 aufweisen.

Weitere erfindungsgemäß geeignete Kunststoffdispersionen sind zum anderen die im Bauwesen zur Anwendung gelangenden wäßrigen Dispersionen auf Basis von Polyvinylacetat. Beispielsweise seien Ethylen-Vinylacetat-Copolymere und Vinyllaurat-Vinylacetat-Copolymere genannt (Plaste und Kautschuk, 17, 162 (1970)).

Bevorzugte Kunststoffdispersionen für die erfindungsgemäßen Laminatverbände sind stabil gegenüber Kalzium- und/oder Sulfationen. Besonders bevorzugte Kunststoffdispersionen für die erfindungsgemäßen Laminatverbände sind in Wasser dispergierbare Polyurethane mit im wesentlichen linearem Molekularaufbau, gekennzeichnet durch

a) endständige Polyalkylenoxid-Polyetherketten mit einem Gehalt an Ethylenoxid-Einheiten von 0,5 bis 10 Gew.-%, bezogen auf das gesamte Polyurethan, und

b) einen Gehalt an $=N^{\oplus}=$, $=S^{\oplus}=$, $-COO^{\ominus}$ oder $-SO_3^{\ominus}$-Gruppen von 0,1 bis 15 Milliäquivalent pro 100 g.

Die erfindungsgemäßen Laminatverbände bestehen zumindest aus einer Gipslage und einer Kunststoff-

4

harzlage, wobei die äußere Lage bevorzugt eine Gipslage ist.

Im allgemeinen bestehen die erfindungsgemäßen Laminatverbände alternierend aus 2 bis 7, bevorzugt 3 bis 5, Gips- und Kunststoffharzlagen.

Bei den erfindungsgemäßen Laminatverbänden sind zumindest die Gipslagen mit einer Kunststoffdispersion ausgerüstet. Im Rahmen der vorliegenden Erfindung ist es jedoch auch möglich, sowohl die Gipslage als auch die Kunststofflage mit der wäßrigen Kunststoffdispersion zu imprägnieren.

Die Imprägnierungsrate beträgt im allgemeinen 1 bis 10 Gew.-%, bevorzugt 2 bis 5 Gew.-%, bezogen auf die jeweilige Lage.

Es wurde auch ein Verfahren zur Herstellung der erfindungsgemäßen Laminatverbände aus getrennten Lagen Gips und Kunststoffharz, die jeweils auf einem Trägermaterial angebracht sind, gefunden, das dadurch gekennzeichnet ist, daß man zumindest die zur Anwendung kommenden Gipslagen und gegebenenfalls die Kunststoffharzlagen mit einer wäßrigen Kunststoffdispersion behandelt und dann die Gipslagen und die Kunststoffharzlagen alternierend übereinander anbringt.

Die erfindungsgemäßen Laminatverbände können beispielsweise wie folgt hergestellt werden:

Die Gipslagen auf dem Trägermaterial (Gipsbinden) werden mit den wäßrigen Kunststoffdispersionen getränkt, um dann sofort ohne weiteres Auspressen feucht über den zu stützenden Körper gewickelt zu werden, der gegebenenfalls vorher mit einem Untergrundmaterial abgedeckt worden ist. Nachdem man eine Gipslage aufgewickelt hat, werden auf dem feuchten Gips die Kunststoffharzlagen (Kunststoffbinden) aufgebracht. Diese Kunststoffbinde wird in Wasser getaucht oder gegebenenfalls mit der wäßrigen Kunststoffdispersion imprägniert.

In der beschriebenen Weise können alternierend die weiteren Gips- und Kunststoffharzlagen gewickelt werden.

Bei dem Verfahren zur Herstellung des erfindungsgemäßen Laminatverbandes imprägniert man die Kunststoffharzlage und die Gipslage mit einer 1 bis 50 gew.-%igen, bevorzugt 2 bis 20 gew.-%igen, wäßrigen Kunststoffdispersion.

Es ist möglich, daß die wäßrige Kunststoffdispersion weitere wasserlösliche organische Lösungsmittel wie Alkohole (z.B. Methanol oder Ethanol) oder Aceton in Mengen von 1 bis 20 Gew.-%, bevorzugt 2 bis 10 Gew.-%, enthält.

Die Imprägnierung wird im allgemeinen so durchgeführt, daß die Gipslage und/oder die Kunststoffharzlage mit der Kunststoffdispersion gesättigt ist. Eine ausreichende Sättigung wird im allgemeinen durch Eintauchen für 2 bis 10 Sekunden erreicht.

Es ist wesentlich für die vorliegende Erfindung, daß die Aushärtung der Gips- bzw. Kunststoffbinden mit der wäßrigen Kunststoffdispersion erfolgt.

Erfindungsgemäß können 2 bis 7 Lagen aus Gips und Kunststoffharz, jeweils auf einem Trägermaterial, wobei zumindest die Gipslagen mit einer wäßrigen Kunststoffdispersion ausgerüstet sind, zur Herstellung von Laminatverbänden verwendet werden.

Es ist als äußerst überraschend zu bezeichnen, daß die erfindungsgemäßen Laminatverbände im Gegensatz zu den in der EP 128 399 beschriebenen mit wäßrigen Polyurethan(harnstoff)dispersionen imprägnierte Gipsverbände rasch aushärten und überschüssiges Wasser leicht abgeben.

Die erfindungsgemäßen Laminatverbände zeichnen durch ausgezeichnete Wasserfestigkeit, wobei jedoch der Wassertransport nicht unterbunden wird, hohe Elastizität und hohe Bruchsicherheit aus. Bemerkenswert ist, daß bei den erfindungsgemäßen Laminatverbänden ein Ausbrechen und Zerbröseln der Ränder unterbleibt. Im Vergleich zu nichtausgerüsteten Laminatverbänden ist der Verbund zwischen den einzelnen Laminatschichten sehr hoch.

Die erfindungsgemäßen Laminatverbände eignen sich besonders für Stützverbände zur Ruhigstellung von menschlichen oder tierischen Körperteilen.

## Beispiele

In den nachfolgenden Beispielen werden die folgenden Komponenten verwendet:

1. Gipsbinden:

Es werden handelsübliche Gipsbinden (Biplatrix® der Firma Beiersdorf) verwendet, bei denen 500 bis 600 g/m² Gips auf einen Träger aus einem Baumwollgewirke aufgebracht sind.

## 2. Kunststoffharzbinden

Es werden handelsübliche Kunststoffharzbinden verwendet, bei denen ein textiles Gewirke aus Baumwolle mit $60 \pm 10$ g/m² Flächengewicht mit $150 \pm 20$ Gew.-% eines aromatische Isocyanatgruppen aufweisenden Polyurethans, NCO-Gehalt = $18 \pm 2$ Gew.-%, Viskosität (25°C) = 10 000 - 25 000 mPas, beschichtet ist (Deltacast® der Firma Johnson + Johnson GmbH).

## 3. Kunststoffdispersion

### a) Polyurethandispersion (PU-Dispersion)

Die nachfolgend zur Anwendung gelangende PU-Dispersion läßt sich folgendermaßen herstellen: 1632 Teile eines Polyesterdiols aus Hexandiol-1,6, 2,2-Dimethylpropandiol-1,3 und Adipinsäure der OH-Zahl 63 wird bei 100°C im Vakuum von etwa 14 Torr entwässert und nach Zugabe von 85 Teilen eines Polyethermonoalkohols aus n-Butanol, Ethylenoxid und Propylenoxid (im Molverhältnis 83:17) der OH-Zahl 30 (1), mit einem Gemisch aus 244,2 Teilen 3-Isocyanatomethyl-3,5,5-trimethylhexylisocyanat und 185 Teilen Hexandiisocyanat-1,6 versetzt. Die Mischung wird bei 100°C so lange gerührt, bis sie einen Gehalt von 4,6 Gew.-% NCO aufweist. Nach dem Abkühlen auf 50-60°C werden 3200 Teile wasserfreien Acetons zugegeben. In diese acetonische Lösung wird ein Gemisch von 107 Teilen (2-Aminoethyl)-2-aminoethansulfon-saures Natrium und 10 Teilen Hydrazinmonohydrat, gelöst in 260 Teilen Wasser, langsam eingerührt. Nach 10-minütigem Nachrühren werden unter lebhaftem Rühren 2280 teile Wasser langsam eingerührt. Dabei bildet sich eine bläulich-weiße Dispersion des Festkörpers in einer Mischung aus Wasser und Aceton. Nach der destiltativen Entfernung des Acetons bleibt eine wäßrige Dispersion des Festkörpers mit einer Konzentration von 50 %. Die Messung des Teilchendurchmessers mit Hilfe der Lichtstreuung ergibt einen Wert von $200 \pm 20$ nm. Der Festkörper der Dispersion enthält 3,1 % Polyethylenoxidsegmente und 3 m Äquivalent Sulfonatgruppen ($-SO_3^\ominus$) auf 100 g Festkörper.

### b) Polyvinylharzdispersion (EVA-Dispersion)

Handelsübliche wäßrige Dispersion eines Copolymeren aus Vinylacetat und Ethylen; pH = 4; Teilchengröße 0,5 bis 1 µm (Vinnapas®-Dispersion der Wacker-Chemie GmbH, Firmendruckschrift Nr. 3710, 283).

### Beispiel 1 (Herstellung von ausgerüsteten Gipsbinden)

In 1.900 g einer 5 gew.-%igen PU-Dispersion werden nacheinander 14 Gipsbinden 8 cm x 400 cm (Trockengewicht ca. 170 g) jeweils 4 bis 5 Senkungen getaucht, bis keine Luftblasen mehr aufsteigen. Die Binden werden nur schwach ausgedrückt, herausgenommen und dann zu entsprechenden Probekörper nachfolgender Beispiele verarbeitet. Die verbleibende Dispersion wird untersucht:
Menge: 780 g, Feststoffanteil: 5,65 Gew.-%.

Dies bedeutet, daß die 14 Gipsbinden nach der Behandlung mit der wäßrigen PU-Dispersion 51 g Feststoffanteil der Kunststoffdispersion aufgenommen haben, welches einem Imprägnierungsgrad von 2,3 % entspricht.

### Beispiel 2

Es werden nachfolgende Probekörper gewickelt:
Innendurchmesser:  87 mm
Breite:  90 mm.

Probekörper A:

1. Lage  Eine Gipsbinde 10 cm x 300 cm wird 5 Sekunden in Wasser getaucht und dreimal um eine entsprechende Hülse (Durchmesser: 87 mm) gewickelt.

2. Lage:  Eine Kunststoffharzbinde 10 cm x 300 cm wird 5 Sekunden unter Ausdrücken in Wasser getaucht und zweimal um die vorhandene Gipslage gewickelt.

3. Lage:  Eine weitere Gipsbinde 10 cm x 300 cm wird 5 Sekunden in Wasser getaucht und dreimal um die vorhandene Kunststoffharzbinde gewickelt. Nach Aushärtung und Entfernung der Hülse erhält man einen Formkörper, der in Radialrichtung eine Bruchfestigkeit von 15,5 N/cm aufweist.

(Bestimmt mit einer Maschine der Fa. Zwick Nr. 1484, Probekörper zwischen Platten, Maximalwert bei 25 mm/Min Vorschub.)

Probekörper B:

1. Lage wie A, jedoch statt Wasser 10 gew.-%ige EVA-Dispersion.
2. Lage wie A.
3. Lage wie A, jedoch statt Wasser 10 gew.%ige EVA-Dispersion.
Bruchfestigkeit: 19,1 N/cm (Bestimmung wie A).

Probekörper C:

1. Lage wie A, jedoch statt Wasser 10 gew.-%ige PU-Dispersion.
2. Lage wie A.
3. Lage wie A, jedoch statt Wasser 10 gew.-%ige PU-Dispersion.
Bruchfestigkeit: 19,2 N/cm (Bestimmung wie A).

Beispiel 3

Es wurden nachfolgende Probekörper gewickelt:
Innendurchmesser:     100 mm
Breite:               90 mm

Probekörper A:

1. Lage:     Eine Gipsbinde 8 cm x 400 cm wird 5 Sekunden in Wasser getaucht und dreimal um eine entsprechende Hülse (Druchmesser: 10 cm) gewickelt.
2. Lage:     Eine Kunstharzbinde 7,5 cm x 300 cm wird 5 Sekunden unter Ausdrücken in Wasser getaucht und zweimal um die Gipslage gewickelt.
3. Lage:     Eine Gipsbinde 8 cm x 400 cm wird 5 Sekunden in Wasser getaucht und dreimal um die vorhandene Kunstharzbinde gewickelt.

Nach Aushärtung und Entfernen der Hülse erhält man einen Formkörper, der in Radialrichtung eine Bruchfestigkeit von 12,2 N/cm aufweist. (Bestimmung mit einer Maschine der Fa. Zwick Nr. 1484, Probekörper zwischen Platten, Maximalwert bei 25 mm/Min Vorschub.)

Probekörper B:

1. Lage wie A, jedoch statt Wasser eine 5 gew.-%ige PU-Dispersion.
2. Lage wie A.
3. Lage wie A, jedoch statt Wasser eine 5 gew.-%ige PU-Dispersion.
Bruchfestigkeit: 13,6 N/cm (Bestimmung wie A).

Probekörper C:

1. Lage wie B,
2. Lage wie A, jedoch statt Wasser ebenfalls eine 5 gew.-%ige PU-Dispersion.
3. Lage wie B.
Bruchfestigkeit: 14,6 N/cm (Bestimmung wie A).

Beispiel 4

Es wurden nachfolgende Probekörper gewickelt:
Innendurchmesser:     100 mm
Breite:               200 mm
Probekörper A:     wie Probekörper A, Beispiel 3, jedoch unter Verwendung einer Gipsbinde 10 cm x 300 cm
Probekörper B:     wie Probekörper B, Beispiel 3, jedoch unter Verwendung einer Gipsbinde 10 cm x 300 cm

An den Probekörpern wurde die Maximalbelastung (Bruch bzw. Delaminierung) in Richtung der Längsachse (= stehend) bestimmt:
Probekörper A: 12 kp
Probekörper B: 20 kp.

Beispiel 5

Es wurden nachfolgende Probekörper gewickelt:
Innendurchmesser: 87 mm
Breite: 100 mm

Probekörper A:

1. Lage: Eine Kunststoffharzbinde 10 cm x 300 cm wird 5 Sekunden unter Ausdrücken in Wasser getaucht und dreimal um eine entsprechende Hülse (Durchmesser 87 mm) gewickelt.

2. Lage: Eine Gipsbinde 10 cm x 200 cm (Beschichtungshöhe: $510 \pm 20$ g/m² Gips) wird 5 Sekunden in Wasser getaucht und viermal um die vorhandene Kunstharzlage gewickelt.

Probekörper B:

1. Lage: wie A, jedoch statt Wasser 5 gew.-%ige PU-Dispersion
2. Lage: wie A, jedoch statt Wasser 5 gew.-%ige PU-Dispersion
Nach Aushärtung und Entfernung der Hülse erhält man je einen Formkörper, der in seiner Radialrichtung eine Bruchfestigkeit von
11,4 N/cm    bei A    und
12,4 N/cm    bei B    aufweist.
(Bestimmung mit einer Maschine der Fa. Zwick, Nr. 1484; Probekörper zwischen Platten, Maximalwert bei 25 mm/Min. Vorschub).

**Patentansprüche**

**Patentansprüche für folgende Vertragsstaaten : AT, BE, CH, DE, FR, GB, IT, LI, LU, NL, SE**

1. Wasserfeste mehrlagige Stützverbände aus alternierenden Lagen von
(a) Gips/Trägermaterial-Laminat und
(b) wasserhärtendem Kunststoffharz/Trägermaterial-Laminat, dadurch gekennzeichnet, daß vor der Anfertigung des mehrlagigem Stützverbandes zumindest das Laminat (a) mit einer
(c) wässerigen Kunststoffdispersion getränkt wird.
2. Stützverbände nach Anspruch 1, dadurch gekennzeichnet, daß als wasserhärtendes Kunststoffharz in (b) wasserhärtendes Polyurethan oder wasserhärtendes Polyvinylharz verwendet wird.
3. Stützverbände nach Ansprüchen 1 und 2, dadurch gekennzeichnet, daß man eine Kunststoffdispersion (c) verwendet, die gegenüber Calciumionen stabil ist.
4. Stützverbände nach Ansprüchen 1-3, dadurch gekennzeichnet, daß man als Trägermaterial für die Laminate (a) und (b) textiles Gewebe oder Gewirke und/oder Synthesefasern und/oder Glasfasern verwendet.
5. Stützverbände gemäß Ansprüchen 1-4, dadurch gekennzeichnet, daß man als äußere Lage eine Gipslage verwendet.
6. Stützverbände nach Ansprüchen 1-5, dadurch gekennzeichnet, daß man alternierend 3-7 Lagen (a) und (b) verwendet.
7. Stützverbände gemäß Ansprüchen 1-6, dadurch gekennzeichnet, daß die jeweilige Lage (a) und/oder (b) mit mindestens 1-10 Gew.-% bezogen auf die jeweilige Lage mit der Kunststoffdispersion (c) getränkt wird.
8. Verfahren zur Herstellung von wasserfesten mehrlagigen Stützverbänden aus getrennten Lagen von
(a) Gips/Trägermaterial-Laminat und
(b) wasserhärtendem Kunststoffharz/Trägermaterial-Laminat, dadurch gekennzeichnet, daß man zumindest die zur Anwendung kommenden Lagen des (a) Gips/Trägermaterial-Laminats mit einer
(c) wässerigen Kunststoffdispersion tränkt und anschließend die beiden Laminate alternierend übereinander anbringt.
9. Verfahren nach Anspruch 8, dadurch gekennzeichnet, daß man eine 10-50 Gew.-%ige Kunststoffdisper-

sion (c) verwendet.

**Patentansprüche für folgende Vertragsstaaten : GR, ES**

1. Verfahren zur Herstellung von wasserfesten mehrlagigen Stützverbänden aus getrennten Lagen von
(a) Gips/Trägermaterial-Laminat und
(b) wasserhärtendem Kunststoffharz/Trägermaterial-Laminat, dadurch gekennzeichnet, daß man zumindest die zur Anwendung kommenden Lagen des (a) Gips/Trägermaterial-Laminats mit einer
(c) wässerigen Kunststoffdispersion tränkt und anschließend die beiden Laminate alternierend übereinander anbringt.

2. Verfahren nach Anspruch 1, dadurch gekennzeichnet, daß man eine 10-50 Gew.-%ige Kunststoffdispersion (c) verwendet.

## Claims

**Claims for the following Contracting States : BE, FR, GB, IT, NL, SE**

1. Water-resistant multi-layer supporting dressings consisting of alternating layers of -
(a) gypsum/carrier material laminate and
(b) synthetic resin/carrier material laminate which hardens in water, characterised in that, before the preparation of the multi-layer supporting dressing, at least the laminate (a) is impregnated with
(c) an aqueous synthetic dispersion.

2. Supporting dressings according to Claim 1, characterised in that polyurethane which hardens in water or polyvinyl resin which hardens in water is used in (b) as the synthetic resin which hardens in water.

3. Supporting dressings according to Claims 1 and 2, characterised in that a synthetic dispersion (c) which is stable towards calcium ions is used.

4. Supporting dressings according to Claims 1-3, characterised in that a textile woven fabric or knitted fabric and/or synthetic fibres and/or glass fibres is used as the carrier material for the laminates (a) and (b).

5. Supporting dressings according to Claims 1-4, characterised in that a gypsum layer is used as the outer layer

6. Supporting dressings according to Claims 1-5, characterised in that 3-7 layers (a) and (b) are used in alternation.

7. Supporting dressings according to Claims 1-6, characterised in that the particular layer (a) and/or (b) is impregnated with at least 1-10% by weight, based on the particular layer, of the synthetic dispersion (c).

8. Process for the preparation of water-resistant multi-layer supporting dressings of separate layers of
(a) gypsum/carrier material laminate and
(b) synthetic resin/carrier material laminate which hardens in water, characterised in that at least the layers used of (a) gypsum/carrier material laminate are impregnated with
(c) an aqueous synthetic dispersion and then the two laminates are applied on top of one another in alternation.

9. Process according to Claim 8, characterised in that a 10-50% strength by weight synthetic dispersion (c) is used.

**Claims for the following Contracting States : GR, ES**

1. Process for the preparation of water-resistant multi-layer supporting dressings of separate layers of
(a) gypsum/carrier material laminate and
(b) synthetic resin/carrier material laminate which hardens in water, characterised in that at least the layers used of (a) gypsum/carrier material laminate are impregnated with
(c) an aqueous synthetic dispersion and then the two laminates are applied on top of one another in alternation.

2. Process according to Claim 1, characterised in that a 10-50% strength by weight synthetic dispersion (c) is used.

**Revendications**

**Revendications pour les Etats contractants suivants : BE, FR, GB, IT, NL, SE**

1. Bandage de soutien à plusieurs couches, résistant à l'eau, composé de couches alternées de
(a) aggloméré plâtre/matériau de support et
(b) aggloméré de résine synthétique durcissant à l'eau/matériau de support, caractérisé en ce que, avant la préparation du bandage de soutien à plusieurs couches, au moins l'aggloméré (a) est imprégné
(c) d'une dispersion aqueuse de matière synthétique.

2. Bandage de support selon la revendication 1, caractérisé en ce que l'on utilise un polyuréthanne durcissant à l'eau ou une résine de polyvinyle durcissant à l'eau comme résine synthétique dans (b).

3. Bandage de soutien selon les revendications 1 et 2, caractérisé en ce que l'on utilise une dispersion de matière synthétique (c) qui est stable vis-à-vis des ions calcium.

4. Bandage de soutien selon les revendications 1 - 3, caractérisé en ce que l'on utilise comme matériau de support pour les agglomérés (a) et (b) des tissus ou des tricots textiles et/ou des fibres de synthèse et/ou des fibres de verre.

5. Bandage de soutien selon les revendications 1 - 4, caractérisé en ce que l'on utilise une couche de plâtre comme couche extérieure.

6. Bandage de soutien selon les revendications 1 - 5, caractérisé en ce que l'on utilise 3 à 7 couches (a) et (b) alternées.

7. Bandage de soutien selon les revendications 1 - 6, caractérisé en ce que chaque couche (a) ou (b) est imprégnée de la dispersion de matière synthétique (c) à raison d'au moins 1 à 10% en poids par rapport à la couche concernée.

8. Procédé pour la fabrication de bandages de soutien à plusieurs couches résistant à l'eau composés de couches séparées de
(a) aggloméré plâtre/matériau de support
(b) aggloméré de résine synthétique durcissant à l'eau/matériau de support, caractérisé en ce qu'au moins les couches utilisées de (a) aggloméré plâtre/matériau de support sont imprégnées d'une
(c) dispersion aqueuse de matière synthétique et qu'on superpose ensuite les deux agglomérés de manière alternée.

9. Procédé selon la revendication 8, caractérisé en ce que l'on utilise une dispersion de matière synthétique (c) à 10 - 50% en poids.

**Revendications pour les Etats contractants suivants : GR, ES**

1. Procédé pour fabriquer des bandages de support à plusieurs couches, résistant à l'eau, composés de couches séparées de
(a) aggloméré plâtre/matériau de support et
(b) aggloméré résine synthétique durcissant à l'eau/matériau de support, caractérisé en ce qu'au moins les couches utilisées de (a) aggloméré plâtre/matériau de support sont imprégnées d'une
(c) dispersion aqueuse de matière synthétique et qu'on superpose ensuite les deux agglomérés de manière alternée.

2. Procédé selon la revendication 1, caractérisé en ce que l'on utilise une dispersion (c) à 10 - 50% en poids.